# EUROPÄISCHE PATENTANMELDUNG

(11) **EP 1 626 264 A1**
(43) Veröffentlichungstag der Anmeldung: **15.02.2006**
(21) Anmeldenummer: 04018999.5
(22) Anmeldetag: 11.08.2004
(51) Int. Cl.: G01N 1/34, G01N 33/06

(54) **Verfahren zur Bestimmung des Fettgehalts organischer Proben und Verfahren zum Extrahieren von Fetten aus organischen Proben**

(71) Anmelder: Büchi Labortechnik AG, CH-9230 Flawil (CH)
(72) Erfinder: Lüscher, Heinz, CH-8057 Zürich (CH); Kheradmandan, Sohrab, CH-8041 Zürich (CH); Ziolko, Thomas, CH-9325 Roggwil TG (CH)
(74) Vertreter: Hepp, Dieter

(57) **Zusammenfassung**

Die vorliegende Erfindung betrifft ein Verfahren zur Bestimmung des Fettgehalts einer organischen Probe (1) mittels Spektroskopie, wobei ein Fettanteil vor der spektroskopischen Messung aus der Probe (1) extrahiert wird. Mindestens ein Teil des extrahierten Fettanteils wird anschliessend spektroskopisch analysiert. Besonders bevorzugt ist eine Messung mittels IR-Spektroskopie, insbesondere FT-NIR-Spektroskopie. Der Fettgehalt der organischen Probe wird vorzugsweise mit einem organischen Lösungsmittel aus der Probe extrahiert und mit einem Alkan verseift.

## Beschreibung

Die vorliegende Erfindung betrifft ein Verfahren zur Bestimmung des Fettgehalts von organischen Proben und ein Verfahren zum Extrahieren von Fetten aus organischen Proben sowie eine Anordnung zum Bestimmen des Fettgehalts von organischen Proben gemäss dem Oberbegriff der unabhängigen Patentansprüche.

Es ist bekannt, den Fettgehalt einer organischen Probe mittels spektroskopischen Verfahren zu bestimmen. Spektroskopische Verfahren sind äusserst schnell und präzise. Innert wenigen Minuten kann der Fettgehalt einer Probe abgelesen werden. Ausserdem ist es möglich, die Anteile von gesättigten und ungesättigten Fettsäuren oder von cis- und trans-Fettsäuren anzugeben.

Spektroskopische Verfahren haben jedoch den Nachteil, dass das Gerät für jede Probenart geeicht werden muss, es ist z.B. nicht möglich, den Fettgehalt von Käse und jenen von Fleischprodukten mit der gleichen Eichung zu bestimmen. Die Eichung ist aufwändig und fehleranfällig. Spektroskopische Messmethoden können deshalb mit Vorteil in Bereichen eingesetzt werden, wo immer gleichartige Proben vermessen werden, z.B. in Molkereien.

Es ist Aufgabe der vorliegenden Erfindung, ein spektroskopisches Verfahren bereitzustellen, mit welchem der Fettgehalt von organischen Proben unabhängig von der Art der Probe zuverlässig bestimmt werden kann.

Diese Aufgabe wird gelöst mit einem Verfahren zum Bestimmen des Fettgehalts von organischen Proben und einem Verfahren zum Extrahieren von Fetten aus organischen Proben sowie einer Anordnung zum Bestimmen des Fettgehalts von organischen Proben gemäss dem kennzeichnenden Teil der unabhängigen Patentansprüche.
Beim Verfahren zur Bestimmung des Fettgehalts einer organischen Probe mittels Spektroskopie wird ein Fettanteil vor der spektroskopischen Messung aus der Probe extrahiert. Von diesem Fettanteil wird zumindest ein Teil anschliessend spektroskopisch analysiert.

Bevorzugt wird NIR-Spektroskopie, insbesondere FT-NIR-Spektroskopie, verwendet, insbesondere in einem Wellenlängenbereich von 780 bis 2500 nm.

Alternativ kann jede Art von Molekül-Spektroskopie verwendet werden. Dies umfasst z.B. Massenspektroskopie, NMR-Spektroskopie, Ramanspektroskopie oder allgemeine IR-Spektroskopie. Andere Spektroskopische Verfahren sollen ebenfalls nicht ausgeschlossen werden.

Mit dem vorliegenden Verfahren kann der Gesamtgehalt an Fett, der Anteil an gesättigten und ungesättigten Fettsäuren, der Anteil an Buttersäuren, sowie der Anteil an Cis- oder Trans-Fettsäuren bestimmt werden. Je nach Anwendung ist es wünschenswert, alle diese Anteile oder nur einzelne davon zu bestimmen.

Beim vorliegenden Verfahren wird das Fett vorzugsweise mit einem organischen Lösungsmittel aus der Probe extrahiert. Durch die Wahl eines Lösungsmittels mit hohem Siedepunkt kann sichergestellt werden, dass im wesentlichen der gesamte Fettanteil aus der Probe gelöst wird. Der extrahierte Fettanteil, welcher z.B. als Triglycerid vorliegen kann, wird in einem alkalischen Aufschluss vorzugsweise gleichzeitig verseift. Anschliessend werden die verseiften Fette in die entsprechenden freien Fettsäuren überführt. Vorzugsweise wird mit einem hochsiedenden Lösungsmittel bei einer Temperatur von mindestens 100°C extrahiert.

Es ist vorteilhaft, das Reaktionsprodukt zum selektiven Analysieren in seine einzelnen Bestandteile aufzutrennen. Dadurch können die Fettsäuren vom Lösungsmittel und voneinander getrennt werden. Wenn ausserdem ein hochsiedendes polares Lösungsmittel verwendet wird, ist auch eine Trennung von weiteren unerwünschten Produkten wie Fettalkoholen, Farbstoffen oder ReaktionsNebenprodukten möglich. Als hochsiedend werden Lösungsmittel mit einem Siedepunkt von über 100°C bezeichnet. Bevorzugt ist ein Lösungsmittel mit einer Siedetemperatur zwischen 110 und 120°C.

Als hochsiedendes Lösungsmittel wird beispielsweise n-Butylalkohol verwendet. Grundsätzlich sind längerkettige Alkanole aufgrund ihrer schlechten Löslichkeit in Wasser geeignet. Als längerkettig werden Alkanole mit einer Kettenlänge von vier oder mehr C-Atomen betrachtet. Dem Fachmann sind die Löslichkeiten der Lösungsmittel bekannt und er wird entsprechend ein geeignetes Lösungsmittel auswählen.

Als Base zum Verseifen der aus der Probe herausgelösten Fettanteile sind insbesondere Alkalihydroxide wie z.B. Kaliumhydroxid geeignet. Denkbar ist z.B. auch die Verwendung von Natriumhydroxid.

Durch das Beigeben einer wässrigen, sauren Salzlösung nach dem Verseifen ergibt sich eine Trennung in eine wässrige und in eine darüber liegende organische Phase, welche die freien Fettsäuren enthält. Zum Analysieren des Reaktionsprodukts und zum Bestimmen des gesamten Gehalts an Fettsäuren wird eine Probe aus der organischen Schicht entnommen.

Alternativ kann die Probenaufbereitung folgende Schritte umfassen:
- Aufschluss der Probe mit einer alkalischen Lösung eines niedermolekularen Alkohols, insbesondere Methanol und/oder Ethanol
- Hydrolyse mit einer sauren, wässrigen Lösung
- Extraktion der freien Fettsäuren mit einem unpolaren organischen Lösungsmittel, vorzugsweise einem Alkan, insbesondere einem längerkettigem Alkan, vorzugsweise n-Hexan.

Als niedermolekularer Alkohol wird ein Alkohol mit einer Kettenlänge von weniger als vier C-Atomen betrachtet. Die Ketten können auch verzweigt sein. Bevorzugt ist eine Kettenlänge von ein bis zwei C-Atomen. Wesentlich ist die gute Löslichkeit des Alkohols in Wasser, wie sie bei niedermolekularen Alkoholen gegeben ist.

Als längerkettiges Alkan wird ein Alkan mit einer Kettenlänge von fünf oder mehr C-Atomen betrachtet. Diese werden aufgrund ihrer schlechten Löslichkeit in Wasser gewählt.

Der Aufschluss der Probe findet vorzugsweise während ca. 30 bis 40 Minuten statt.

Selbstverständlich sind auch andere Verfahren der Probenaufbereitung für die vorliegende Erfindung geeignet. Andere Verfahren mit organische Lösungsmitteln oder die Verwendung von flüssigem Kohlendioxid zur Extraktion der Fette sind ebenfalls denkbar.

Das Verfahren zum Extrahieren von Fetten aus organischen Proben umfasst folgende Schritte:
- Aufschluss der Probe mit einer alkalischen Lösung eines niedermolekularen Alkohols, insbesondere Methanol und/oder Ethanol
- Hydrolyse mit einer sauren, wässrigen Lösung
- Extraktion der freien Fettsäuren mit einem unpolaren organischen Lösungsmittel, vorzugsweise einem Alkan, insbesondere mit einem längerkettigem Alkan, vorzugsweise n-Hexan.

Im Unterschied zum vorangehend beschriebenen Verfahren ist bei der Verwendung eines niedermolekularen Alkohols das Beifügen eines unpolaren organischen Lösungsmittels notwendig, damit eine Phasentrennung auftritt. Erst nach der Phasentrennung können die Fettsäuren lokalisiert und extrahiert werden.

Die Vorteile der vorliegenden Erfindung sind, dass der Fettgehalt einer organischen Probe spektroskopisch bestimmt werden kann, ohne dass das Gerät zuvor aufwändig geeicht werden muss. Ausserdem ist das Verfahren relativ schnell und zuverlässig und daher für den Routinegebrauch geeignet.

Besonders vorteilhaft ist die Erfindung, wenn der Fettanteil immer auf die gleiche Art, d.h. mit den gleichen Lösungsmitteln, insbesondere unter Verwendung des gleichen Extraktionsverfahrens, extrahiert wird. Dadurch genügt es, das Spektroskopie-Gerät für diese Lösungsmittel, insbesondere unter Verwendung des gleichen Extraktionsverfahrens einmalig zu eichen. Die Eichung kann dazu vorteilhaft elektronisch von einem bereits geeichten Gerät auf ein neues Gerät übertragen werden, was die Anwenderfreundlichkeit weiter erhöht.

Alternativ können mehrere Verfahren zur Extraktion der Fette verwendet werden, es ist jedoch notwendig, das Gerät für jedes Extraktionsverfahren separat zu eichen.

Im folgenden wird die Erfindung anhand von Figuren und Ausführungsbeispielen erläutert. Es zeigen:
- Fig. 1:: Das erfindungsgemässe Verfahren nach einer ersten Ausführungsform
- Fig. 2:: Das erfindungsgemässe Verfahren nach einer zweiten Ausführungsform

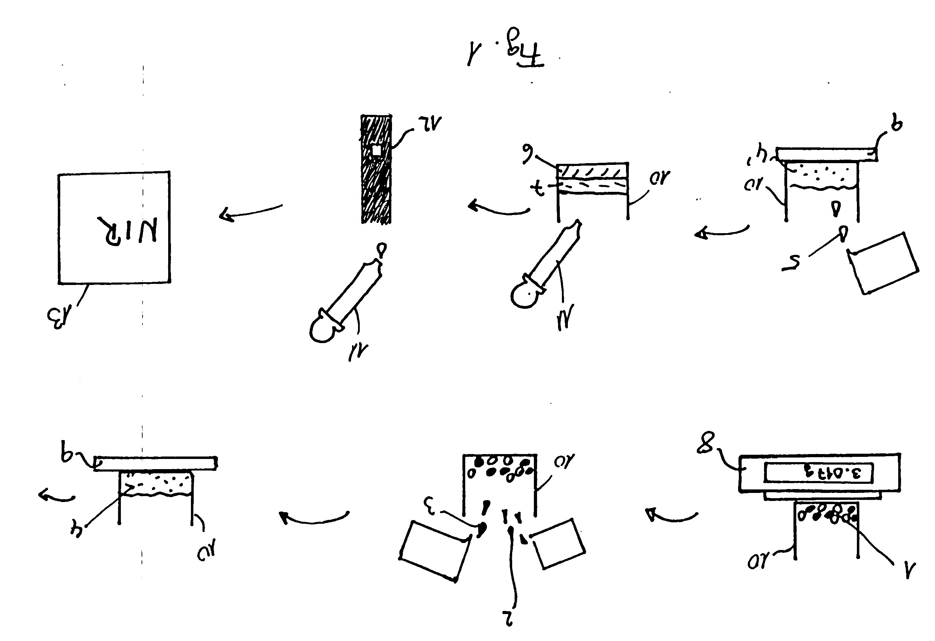

In einer ersten Ausführungsform gemäss Fig. 1 werden 0.5 bis 10 g einer homogenisierten Lebensmittelprobe 1 werden auf einer Waage 8 auf 1 mg genau gewogen. Die Einwaage hängt vom Fettgehalt der Lebensmittelprobe ab. Je grösser der Fettgehalt ist, desto kleiner wird die Einwaage. Eine durchschnittliche Probe beträgt ca. 3 g.

Die gewogene Lebensmittelprobe wird mit 1.5 bis 2 g Kaliumhydroxid-Plättchen 2 und 50 ml Butanol 3 höchster Reinheit vermengt. Die Mischung wird während 30 Minuten unter Rühren, z.B. mit einem Magnetrührer 9, bei 150 °C unter Rückfluss erhitzt.

Nach ca. 10 Minuten ist die Probe in der stark alkalischen Butanol-Lösung (pH > 14) aufgelöst, so dass eine Lösung 4 entsteht. Nach Ablauf der 30 Minuten werden 50 ml einer wässrigen sauren Lösung 5 mit einer Kanüle (nicht dargestellt) in das Reaktionsgefäss 10 eingeleitet und während 10 Minuten weitergerührt. Die Temperatur der Lösung liegt während dieser Zeit noch mindestens bei 100°C. Die Lösung wird aber nicht mehr weiter geheizt.

Die wässrige saure Lösung 5 wird aus 325 g Natriumdiydrogenphosphat (NaH₂PO₄) und 50 ml Ameisensäure (HCOOH) hergestellt, indem die beiden Stoffe in 500 ml Wasser gelöst werden und die Lösung anschliessend auf 1 1 aufgefüllt wird. Der pH-Wert dieser Lösung beträgt 3 bis 4.

Wenn nicht mehr gerührt wird, teilt sich die Lösung 4' in zwei Phasen 6 und 7. Die Butanol-Phase 7 schwimmt oben auf, während die wässrige Phase 6 absinkt. Von der Butanol-Phase 6, in der sich die extrahierten Fette befinden, werden ca. 2 bis 4 ml mit einer Pipette in eine Kuvette 12 gegeben. Die Kuvette wird anschliessend in ein NIR-Gerät 13 gegeben, wo der Fettgehalt anhand einer Transmissions-Messung bestimmt wird.

Alternativ könnten 10 ml von der Butanol-Phase 6 mit einer Pipette 11 auf eine Glas-Petrischale (nicht dargestellt) gegeben, mit einem Transflexionskopf (nicht dargestellt) abgedeckt und mittels Transflextion am NIR-Gerät 13 gemessen werden.

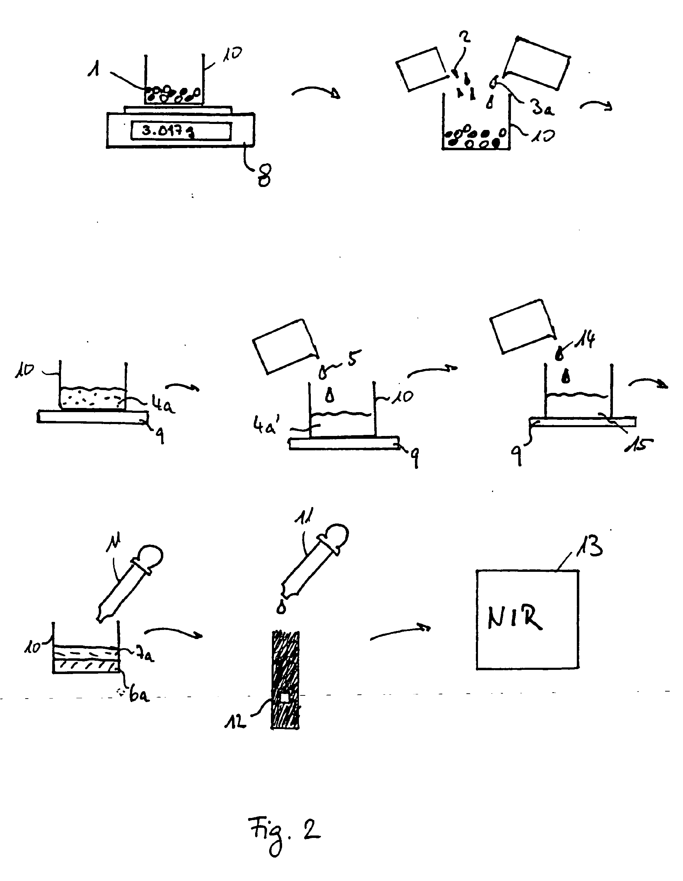

In einer weiteren Ausführungsform gemäss Fig. 2 wird statt n-Butanol 3 Ethanol 3a verwendet und die Lösung während 40 Minuten unter Rühren bei 80 °C unter Rückfluss erhitzt. Nach ca. 20 Minuten löst sich die Lebensmittelprobe 1 in der stark alkalischen Ethanol-Lösung (ph ≥ 14), so dass eine Suspension 4a entsteht.

Nach Ablauf der 40 Minuten werden 50 ml wässrige saure Lösung 5 beigefügt und das Gemisch 4a' während 10 Minuten weitergerührt. Das Gemisch wird dazu nicht weiter geheizt, hat aber noch eine Temperatur vom mindestens 50°C. Anschliessend werden bei Raumtemperatur 20 ml n-Hexan 14 hinzugefügt und das Gemisch 15 während 10 Minuten weitergerührt.

Wenn nicht mehr gerührt wird, teilt sich die Mischung in zwei Phasen. Die Hexan-Phase 7a befindet sich oben, während die wässrige Phase 6a nach unten absinkt. Von der Hexan-Phase 7a werden ca. 2-4 ml mit einer Pipette 11 in eine Kuvette 12 gegeben und der Fettgehalt mittels Transmission im NIR-Gerät 13 bestimmt. Alternativ könnten 10 ml der Hexan-Phase 7a auf eine Glas-Petrischale (nicht dargestellt) gegeben, mit einer Transflexionskopf (nicht dargestellt) abgedeckt und in einer Transflextions-Messung am NIR-Gerät 13 gemessen werden.

## Patentansprüche

1. Verfahren zur Bestimmung des Fettgehalts einer organischen Probe (1) mittels Spektroskopie, wobei ein Fettanteil vor der spektroskopischen Messung aus der Probe (1) extrahiert wird und wenigstens ein Teil des extrahierten Fettanteils spektroskopisch analysiert wird.

2. Verfahren nach Anspruch 1, **dadurch gekennzeichnet, dass** NIR-Spektroskopie verwendet wird.

3. Verfahren nach Anspruch 2, **dadurch gekennzeichnet, dass** eine NIR-Analyse in einem Wellenlängenbereich von 780 bis 2500 nm vorgenommen wird.

4. Verfahren nach Anspruch 1, **dadurch gekennzeichnet, dass** Molekül-Spektroskopie verwendet wird.

5. Verfahren nach einem der Ansprüche 1 bis 4, **dadurch gekennzeichnet, dass** der Gesamtgehalt an Fett, der Anteil an gesättigten und ungesättigten Fettsäuren, der Anteil an Buttersäuren, und/oder der Anteil an Cis- oder Trans-Fettsäuren bestimmt wird.

6. verfahren nach einem der Ansprüche 1 bis 5, **dadurch gekennzeichnet, dass** der Fettanteil mit einem organischen Lösungsmittel (3;3a) extrahiert und der extrahierte Fettanteil verseift wird.

7. Verfahren nach Anspruch 6, **dadurch gekennzeichnet, dass** die Extraktion mit einem hochsiedenden Lösungsmittel (3;3a) bei einer Temperatur von mindestens 100°C vorgenommen wird und der extrahierte Fettanteil verseift wird, und anschliessend die Umwandlung in die entsprechenden Fettsäuren stattfindet.

8. Verfahren nach einem der Ansprüche 1 bis 7, **dadurch gekennzeichnet, dass** ein hochsiedendes Lösungsmittel (3;3a) mit polarem Verhalten verwendet wird.

9. Verfahren nach einem der Ansprüche 1 bis 8, **dadurch gekennzeichnet, dass** als hochsiedendes Lösungsmittel ein Alkanol mit mindestens fünf C-Atomen, insbesondere n-Butylalkohol (3), verwendet wird.

10. Verfahren nach einem der Ansprüche 1 bis 9, **dadurch gekennzeichnet, dass** als Base zum Verseifen der aus der Probe herausgelösten Fettanteile Kaliumhydroxid (2) verwendet wird.

11. Verfahren nach einem der Ansprüche 1 bis 10, **dadurch gekennzeichnet, dass** nach dem Verseifen ein saures Salz dem Reaktionsprodukt zur Umwandlung in Fettsäuren beigefügt wird, so dass eine obere organische Phase (7;7a) und eine untere wässrige Phase (6;6a) gebildet werden.

12. Verfahren nach einem der Ansprüche 1 bis 11, **dadurch gekennzeichnet, dass** das Reaktionsprodukt zum selektiven Analysieren in seine einzelnen Bestandteile aufgetrennt wird.

13. Verfahren nach einem der Ansprüche 1 bis 5, **dadurch gekennzeichnet, dass** die Probenaufbereitung folgende Schritte umfasst:
- Aufschluss der Probe mit einer alkalischen Lösung eines niedermolekularen Alkohols, insbesondere Methanol und/oder Ethanol (3a)
- Hydrolyse mit einer sauren, wässrigen Lösung (5)
- Extraktion der freien Fettsäuren mit einem unpolaren organischen Lösungsmittel, vorzugsweise einem Alkan, insbesondere einem längerkettigem Alkan, vorzugsweise n-Hexan (14).

14. Verfahren nach Anspruch 12, **dadurch gekennzeichnet, dass** der Aufschluss der Probe während ca. 30 bis 40 Minuten stattfindet.

15. Verfahren zum Extrahieren von Fetten aus organischen Proben, insbesondere für ein Verfahren zum Bestimmen des Fettgehalts der organischen Probe (1) gemäss einem der Ansprüche 1 bis 6 oder 12 bis 13, umfassend die Schritte
- Aufschluss der Probe mit einer alkalischen Lösung eines niedermolekularen Alkohols, insbesondere Methanol und/oder Ethanol (3)
- Hydrolyse mit einer sauren, wässrigen Lösung (5)
- Extraktion der freien Fettsäuren mit einem unpolaren organischen Lösungsmittel, vorzugsweise einem Alkan, insbesondere mit einem längerkettigem Alkan, vorzugsweise n-Hexan (14).

16. Anordnung zum Bestimmen des Fettgehalts von organischen Proben (1), enthaltend eine Anordnung (8-12) zum Aufschluss der Probe und zur Extraktion eines Fettanteils aus der Probe und eine Anordnung (13) zur spektroskopischen Analyse des extrahierten Fettanteils.
